(19) Europäisches Patentamt
European Patent Office
Office européen des brevets

(11) EP 1 611 073 B1

(12) EUROPÄISCHE PATENTSCHRIFT

(45) Veröffentlichungstag und Bekanntmachung des Hinweises auf die Patenterteilung:
**08.08.2012 Patentblatt 2012/32**

(21) Anmeldenummer: **04722151.0**

(22) Anmeldetag: **20.03.2004**

(51) Int Cl.:
*C07C 45/35* (2006.01) *C07C 51/25* (2006.01)
*C07C 47/22* (2006.01) *C07C 57/04* (2006.01)

(86) Internationale Anmeldenummer:
**PCT/EP2004/002932**

(87) Internationale Veröffentlichungsnummer:
**WO 2004/085362 (07.10.2004 Gazette 2004/41)**

(54) **VERFAHREN DER HETEROGEN KATALYSIERTEN PARTIELLEN GASPHASENOXIDATION VON PROPEN ZU ACROLEIN**

METHOD FOR THE HETEROGENEOUSLY CATALYZED PARTIAL GAS PHASE OXIDATION OF PROPENE INTO ACROLEIN

PROCEDE D'OXYDATION EN PHASE GAZEUSE PARTIELLE, HETEROGENE ET CATALYSEE DU PROPENE EN ACROLEINE

(84) Benannte Vertragsstaaten:
**AT BE BG CH CY CZ DE DK EE ES FI FR GB GR HU IE IT LI LU MC NL PL PT RO SE SI SK TR**

(30) Priorität: **25.03.2003 DE 10313210**
**05.06.2003 US 475795 P**

(43) Veröffentlichungstag der Anmeldung:
**04.01.2006 Patentblatt 2006/01**

(73) Patentinhaber: **BASF SE**
**67056 Ludwigshafen (DE)**

(72) Erfinder:
- **PETZOLDT, Jochen**
**68163 Mannheim (DE)**
- **DIETERLE, Martin**
**68167 Mannheim (DE)**
- **ARNOLD, Heiko**
**Zijin Villas, Nanjing 210014 (CN)**
- **RUPPEL, Wilhelm**
**68163 Mannheim (DE)**
- **MÜLLER-ENGEL, Klaus, Joachim**
**76297 Stutensee (DE)**

(56) Entgegenhaltungen:
**WO-A-00/53556**

EP 1 611 073 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Vorliegende Erfindung betrifft ein Verfahren der heterogen katalysierten partiellen Gasphaserioxidation von Propen zu Acrolein, bei dem man ein Propen, molekularen Sauerstoff und wenigstens ein Inertgas enthaltendes Reaktionsgasausgangsgemisch, das den molekularen Sauerstoff und das Propen in einem molaren Verhältnis $O_2$:$C_3H_6$ ≥1 enthält, in einer Reaktionsstufe mit der Maßgabe über eine Festbettkatalysatorschüttung, deren Aktivmasse wenigstens ein die Elemente Mo, Fe und Bi enthaltendes Multimetalloxid ist, führt, dass

- die Festbettkatalysatorschüttung in zwei räumlich aufeinander folgenden Temperaturzonen A, B angeordnet ist,
- sowohl die Temperatur der Temperaturzone A als auch die Temperatur der Temperaturzone B eine Temperatur im Bereich von 290 bis 380˚C ist,
- die Festbettkatalysatorschüttung aus wenigstens zwei räumlich aufeinanderfolgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt,
- sich die Temperaturzone A bis zu einem Umsatz des Propens von 40 bis 80 mol.-% erstreckt,
- bei einmaligem Durchgang des Reaktionsgasausgangsgemisches durch die gesamte Festbettkatalysatorschüttung der Propenumsatz ≥90 mol.-% und die Selektivität der Acroleinbildung, bezogen auf umgesetztes Propen, ≥90 mol.-% beträgt,
- die zeitliche Abfolge, in der das Reaktionsgasgemisch die Temperaturzonen A, B durchströmt der alphabetischen Abfolge der Temperaturzonen entspricht,
- die Belastung der Festbettkatalysatorschüttung mit dem im Reaktionsgasausgangsgemisch enthaltenen Propen ≥90 Nl Propen/l Festbettkatalysatorschüttung • h beträgt, und
- die Differenz $T^{maxA}$ - $T^{maxB}$, gebildet aus der höchsten Temperatur $T^{maxA}$, die das Reaktionsgasgemisch innerhalb der Temperaturzone A aufweist, und der höchsten Temperatur $T^{maxB}$, die das Reaktionsgasgemisch innerhalb der Temperaturzone B aufweist, ≥0˚C beträgt.

Das vorgenannte Verfahren der heterogen katalysierten partiellen Gasphasenoxidation von Propen zu Acrolein ist z.B. aus der WO 00/53556 prinzipiell bekannt und insbesondere als erste Oxidationsstufe bei der Herstellung von Acrylsäure durch zweistufige katalytische Gasphasenoxidation ausgehend von Propen von Bedeutung. Acrylsäure ist ein bedeutendes Monomer, das als solches oder in Form seiner Alkylester zur Erzeugung von z.B. als Klebstoffen geeigneten Polymerisaten Verwendung findet. Acrolein ist ein bedeutendes Zwischenprodukt.

Neben molekularem Sauerstoff und den Reaktanden enthält das Reaktionsgasausgangsgemisch u.a. deshalb Inertgas, um das Reaktionsgas außerhalb des Explosionsbereiches zu halten.

Eine Zielsetzung einer solchen katalysierten partiellen Gasphasenoxidation von Propen zu Acrolein besteht darin, bei einmaligem Durchgang des Reaktionsgasgemisches durch die Reaktionsstufe unter ansonsten vorgegebenen Randbedingungen eine möglichst hohe Selektivität $S^{AC}$ an Acrolein zu erzielen (das ist die Molzahl von zu Acrolein umgesetztem Propen, bezogen auf die Molzahl an umgesetztem Propen).

Eine weitere Zielsetzung besteht darin, bei einmaligem Durchgang des Reaktionsgasgemisches durch die Reaktionsstufe unter ansonsten vorgegebenen Randbedingungen einen möglichst hohen Umsatz $U^p$ an Propen zu erzielen (das ist die Molzahl von umgesetztem Propen, bezogen auf die Molzahl an eingesetztem Propen).

Ist bei hohem $U^P$ die $S^{AC}$ hoch, entspricht dies einer hohen Ausbeute $A^{AC}$ an Acrolein ($A^{AC}$ ist das Produkt $U^P$ • $S^{AC}$; d.h., die Molzahl von zu Acrolein umgesetztem Propen, bezogen auf die Molzahl an eingesetztem Propen).

Eine weitere Zielsetzung einer solchen heterogen katalysierten partiellen Gasphasenoxidation von Propen zu Acrolein besteht darin, eine möglichst hohe Raum-Zeit-Ausbeute ($RZA^{AC}$) an Acrolein zu erzielen (das ist bei einer kontinuierlichen Verfahrensweise die je Stunde und Volumen der verwendeten Festbettkatalysatorschüttung in Litern erzeugte Gesamtmenge an Acrolein).

Bei gleich bleibender gegebener Ausbeute $A^{AC}$ ist die Raum-Zeit-Ausbeute um so größer, je größer die Belastung der Festbettkatalysatorschüttung der Reaktionsstufe mit Propen ist (darunter wird die Menge an Propen in Normlitern (=Nl; das Volumen in Litern, das die entsprechende Propenmenge bei Normalbedingungen, d.h. bei 25˚C und 1 bar, einnehmen würde) verstanden, die als Bestandteil des Reaktionsgasausgangsgemisches pro Stunde durch einen

Liter an Festbettkatalysatorschüttung geführt wird).

Die DE-A 19948241, die DE-A 19910506 und die WO 00 53556 lehren zwar, dass die vorgenannten Ziele mittels des eingangs beschriebenen Verfahrens der heterogen katalysierten partiellen Gasphasenoxidation von Propen zu Acrolein prinzipiell zu erreichen sind.

Dies ist insofern von Vorteil, als sich eine geringe volumenspezifische Aktivität innerhalb einer Festbettkatalysatorschüttung bzw. Festbettkatalysatorschüttungszone z.B. dadurch erzielen lässt, dass man die eigentlichen Aktivmasse tragenden Katalysatorformkörper mit an Aktivmasse freien inerten Verdünnungsformkörpem verdünnt, wodurch die Festbettkatalysatorschüttung insgesamt kostengünstiger wird.

Nachteilig an den Lehren der DE-A 19945241, der DE-A 19910506 sowie der WO 0053556 ist jedoch, dass sie kein entsprechendes Ausführungsbeispiel aufweisen und somit die konkrete Ausgestaltung einer solchen Verfahrensweise offen lassen.

In ähnlicher Weise lehrt die EP-A 1106598 ein Verfahren der heterogen katalysierten partiellen Gasphasenoxidation von Propen zu Acrolein, bei dem die Festbettkatalysatorschüttung der Reaktionsstufe aus mehreren räumlich aufeinanderfolgenden Festbettkatalysatorschüttungszonen besteht, deren volumenspezifische Aktivität innerhalb einer Festbettkatalysatorschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt und in mehreren Temperaturzonen angeordnet sein kann.

Gemäß der Lehr der EP-A 1106598 ist es dabei zweckmäßig, wenn Festbettkatalysatorschüttungszonen und Temperaturzonen räumlich zusammenfallen.

Eingehende Untersuchungen seitens der Anmelderin haben jedoch ergeben, dass ein räumliches Zusammenfallen von Festbettkatalysatorschüttungszonen und Temperaturzonen für ein optimales Erreichen der verschiedenen angesprochenen Zielsetzungen in der Regel nicht förderlich ist.

Dies ist u.a. darauf zurückzuführen, dass der Übergang von der Temperaturzone A in die Temperaturzone B und der Übergang von einer Festbettkatalysatorschüttungszone in eine andere Festbettkatalysatorschüttungszone entweder als im wesentlichen gleich wirkende oder als im wesentlichen entgegengesetzt wirkende Maßnahmen ergriffen werden.

Werden sie als im wesentlichen gleich wirkende Maßnahmen ergriffen (z.B. Übergang von einer kälteren Temperaturzone A in eine heißere Temperaturzone B und Übergang von einer Festbettkatalysatorschüttungszone mit niederer volumenspezifischer Aktivität in eine Festbettkatalysatorschüttungszone mit höherer volumenspezifischer Aktivität; beide Maßnahmen verfolgen den Zweck die Reaktion zu fördern), so schießt die erzielte Gesamtwirkung im Übergangsbereich häufig über das angestrebte Ziel hinaus und es resultiert z.B. eine Minderung der $S^{AC}$.

Werden sie als im wesentlichen entgegengesetzt wirkende Maßnahmen ergriffen (z.B. Übergang von einer heißeren Temperaturzone A in eine kältere Temperaturzone B und Übergang von einer Festbettkatalysatorschüttungszone mit niederer volumenspezifischer Aktivität in eine Festbettkatalysatorschüttungszone mit höherer volumenspezifischer Aktivität; die erste Maßnahme verfolgt den Zweck, die Reaktion zu mindern, die zweite Maßnahme verfolgt den Zweck, die Reaktion zu fördern), so neutralisieren sich die Einwirkungen im Übergangsbereich häufig und es resultiert ein geminderter $U^p$.

Die Aufgabe der vorliegenden Erfindung bestand daher darin, ein Verfahren der heterogen katalysierten partiellen Gasphasenoxidation von Propen zu Acrolein in einer Mehrzonenanordnung zur Verfügung zu stellen, das die Nachteile der Verfahren des Standes der Technik nicht aufweist.

Demgemäss wurde ein Verfahren der heterogen katalysierten partiellen Gasphasenoxidation von Propen zu Acrolein, bei dem man ein Propen, molekularen Sauerstoff und wenigstens ein Inertgas enthaltendes Reaktionsgasausgangsgemisch, das den molekularen Sauerstoff und das Propen in einem molaren Verhältnis $O_2:C_3H_6 \geq 1$ enthielt, in einer Reaktionsstufe mit der Maßgabe über eine Festbettkatalysatorschüttung, deren Aktivmasse wenigstens ein die Elemente Mo, Fe und Bi enthaltendes Multimetalloxid ist, führt, dass

- die Festbettkatalysatorschüttung in zwei räumlich aufeinanderfolgenden Temperaturzonen A, B angeordnet ist,
- sowohl die Temperatur der Temperaturzone A als auch die Temperatur der Temperaturzone B eine Temperatur im Bereich von 290 bis 380°C ist,
- die Festbettkatalysatorschüttung aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt,
- sich die Temperaturzone A bis zu einem Umsatz des Propens von 40 bis 80 mol.-% erstreckt,

- bei einmaligem Durchgang des Reaktionsgasausgangsgemisches durch die gesamte Festbettkatalysatorschüttung der Propenumsatz ≥90 mol.-% und die Selektivität der Acroleinbildung, bezogen auf umgesetztes Propen, ≥90 mol.-% beträgt,

- die zeitliche Abfolge, in der das Reaktionsgasgemisch die Temperaturzonen A, B durchströmt, der alphabetischen Abfolge der Temperaturzonen entspricht,

- die Belastung der Festbettkatalysatorschüttung mit dem im Reaktionsgasausgangsgemisch enthaltenen Propen ≥90 NI Propen/I Festbettkatalysatorschüttung ◦ h beträgt, und

- die Differenz $T^{maxA}$-$T^{maxB}$, gebildet aus der höchsten Temperatur $T^{maxA}$, die das Reaktionsgasgemisch innerhalb der Temperaturzone A aufweist, und der höchsten Temperatur $T^{maxB}$, die das Reaktionsgasgemisch innerhalb der Temperaturzone B aufweist, ≥0˚C beträgt,

gefunden, das dadurch gekennzeichnet ist, dass der Übergang von der Temperaturzone A in die Temperaturzone B in der Festbettkatalysatorschüttung (räumlich) nicht mit einem Übergang von einer Festbettkatalysatorschüttungszone in eine andere Festbettkatalysatorschüttungszone zusammenfällt.

Unter der Temperatur einer Temperaturzone wird in dieser Schrift die Temperatur des in der Temperaturzone befindlichen Teils der Festbettkatalysatorschüttung bei Ausübung des erfindungsgemäßen Verfahrens, jedoch in Abwesenheit einer chemischen Reaktion verstanden. Ist diese Temperatur innerhalb der Temperaturzone nicht konstant, so meint der Begriff Temperatur einer Temperaturzone hier den (Zahlen)mittelwert der Temperatur der Festbettkatalysatorschüttung längs der Temperaturzone. Wesentlich ist dabei, dass die Temperierung der einzelnen Temperaturzonen im wesentlichen unabhängig voneinander erfolgt.

Da die heterogen katalysierte partielle Gasphasenoxidation von Propen zu Acrolein eine ausgeprägt exotherme Reaktion ist, ist die Temperatur des Reaktionsgasgemisches beim reaktiven Durchgang durch die Festbettkatalysatorschüttung in der Regel von der Temperatur einer Temperaturzone verschieden. Sie liegt normalerweise oberhalb der Temperatur der Temperaturzone und durchläuft innerhalb einer Temperaturzone in der Regel ein Maximum (Heißpunktmaximum) oder fällt von einem Maximalwert ausgehend ab.

In der Regel wird beim erfindungsgemäßen Verfahren die Differenz $T^{maxA}$-$T^{maxB}$ nicht mehr als 80˚C betragen. Erfindungsgemäß bevorzugt beträgt $T^{maxa}$-$T^{maxB}$ ≥3˚C und ≤70˚C. Ganz besonders bevorzugt beträgt $T^{maxA}$-$T^{maxB}$ beim erfindungsgemäßen Verfahren ≥20˚C und ≤60˚C.

Die erfindungsgemäß geforderten Differenzen $T^{maxA}$-$T^{maxB}$ stellen sich bei der Ausübung des erfindungsgemäßen Verfahrens im Fall von eher niederen (≥90 Nl/l • h und < 160 Nl/l • h) Propenbelastungen der Festbettkatalysatorschüttung normalerweise dann ein, wenn einerseits sowohl die Temperatur der Temperaturzone A als auch die Temperatur der Temperaturzone B im Bereich von 290 bis 380˚C liegt und andererseits die Differenz zwischen der Temperatur der Temperaturzone B ($T_B$) und der Temperatur der Temperaturzone A ($T_A$), d.h., $T_B$ - $T_A$, ≤0˚C und ≥-20˚C oder ≥-10˚C, bzw. ≤0˚C und ≥-5˚C, oder häufig ≤0˚C und ≥-3˚C beträgt.

Bei Ausübung des erfindungsgemäßen Verfahrens unter erhöhten Propenbelastungen (≥160 Nl/l • h und ≤300 Nl/l • h bzw. ≤600 Nl/l • h) stellen sich die erfindungsgemäß geforderten Differenzen $T^{maxA}$-$T^{maxB}$ normalerweise dann ein, wenn einerseits sowohl die Temperatur der Temperaturzone A als auch die Temperatur der Temperaturzone B im Bereich von 290 bis 380˚C liegt und $T_B$ - $T_A$ > 0˚C und ≤50˚C, oder ≥5˚C und ≤45˚C, oder ≥10˚C und ≤40˚C, oder ≥15˚C und ≤30˚C, oder ≤35˚C (z.B. 20˚C oder 25˚C) beträgt.

Die vorgenannte Aussage betreffend die Temperaturdifferenzen $T_B$-$T_A$ gilt regelmäßig auch dann, wenn die Temperatur der Temperaturzone A im bevorzugten Bereich von 305 bis 365˚C bzw. im besonders bevorzugten Bereich von 310 bis 340˚C liegt.

Die Propenbelastung der Festbettkatalysatorschüttung kann somit beim erfindungsgemäßen Verfahren z.B. ≥90 Nl/l • h und ≤300 Nl/l • h, oder ≥110 Nl/l • h und ≤280 Nl/l • h, oder ≥130 Nl/l • h und ≤260 Nl/l • h, oder ≥150 Nl/l • h und ≤240 Nl/l • h, oder ≥170 Nl/l • h und ≤220 Nl/l • h, oder ≥190 Nl/l • h und ≤200 Nl/l • h betragen.

Erfindungsgemäß bevorzugt erstreckt sich die Temperaturzone A bis zu einem Umsatz des Propens von 50 bis 70 mol-% bzw. 60 bis 70 mol-%.

Der Arbeitsdruck kann beim erfindungsgemäßen Verfahren sowohl unterhalb vom Normaldruck (z.B. bis zu 0,5 bar) als auch oberhalb von Normaldruck liegen. Typischerweise wird der Arbeitsdruck bei Werten von 1 bis 5 bar, häufig 1 bis 3 bar liegen. Normalerweise wird der Reaktionsdruck 100 bar nicht überschreiten.

Das molare Verhältnis von $O_2$: Propen im Reaktionsgasausgangsgemisch muss erfindungsgemäß ≥1 betragen. Häufig wird es bei Werten > 1 liegen. Üblicherweise wird dieses Verhältnis bei Werten ≤3 liegen. Häufig beträgt das molare Verhältnis von $O_2$: Propen im Reaktionsgasausgangsgemisch erfindungsgemäß 1 bis 2 bzw. 1 bis 1,5.

In der Regel kann der auf den einfachen Durchgang bezogene Propenumsatz beim erfindungsgemäßen Verfahren ≥92 mol-%, oder ≥94 mol-%, oder ≥96 mol-% betragen. Die Selektivität der Acroleinbildung wird dabei regelmäßig

≥92 mol-%, bzw. ≥94 mol-%, häufig ≥95 mol-%, oder ≥96 mol-% bzw. ≥97 mol-% betragen.

Als Quelle für den erforderlichen molekularen Sauerstoff kommt sowohl Luft, als auch an molekularem Stickstoff entreicherte Luft in Betracht.

Als Katalysatoren für die Festbettkatalysatorschüttung des erfindungsgemäßen Verfahrens kommen alle diejenigen in Betracht, deren Aktivmasse wenigstens ein Mo, Bi und Fe enthaltendes Multimetalloxid ist.

Dies sind insbesondere die Multimetalloxidaktivmassen der allgemeinen Formel I der DE-A 19955176, die Multimetalloxidaktivmassen der allgemeinen Formel I der DE-A 19948523, die Multimetalloxidaktivmassen der allgemeinen Formeln I, II und III der DE-A 10101695, die Multimetalloxidaktivmassen der allgemeinen Formeln I, II und III der DE-A 19948248 und die Multimetalloxidaktivmassen der allgemeinen Formeln I, II und III der DE-A 19955168 sowie die in der EP-A 700714 genannten Multimetalloxidaktivmassen.

Ferner eignen sich für die Festbettkatalysatorschüttung die Mo, Bi und Fe enthaltenden Multimetalloxidkatalysatoren die in den Schriften DE-A 10046957, DE-A 10063162, DE-C 3338380, DE-A 19902562, EP-A 15565, DE-C 2380765, EP-A 807465, EP- A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden. Dies gilt insbesondere für die beispielhaften Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1 c aus der EP-A 15565 sowie ein in entsprechender Weise herzustellender Katalysator, dessen Aktivmasse jedoch die Zusammensetzung $Mo_{12}Ni_{6,5}Zn_2Fe_2Bi_1P_{0,0065}K_{0,06}Ox \cdot 10\ SiO_2$ aufweist. Ferner sind hervorzuheben das Beispiel mit der laufenden Nr. 3 aus der DE-A 19855913 (Stöchiometrie: $Mo_{12}Co_7Fe_3Bi_{0,6}K_{0,08}Si_{1,6}Ox$) als Hohlzylindervollkatalysator der Geometrie 5 mm x 3 mm x 2 mm bzw. 5 mm x 2 mm x 2 mm (jeweils Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese eine Hohlzylinder-Geometrie der Ausmaße 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Ebenso eignen sich die Multimetalloxidkatalysatoren und Geometrien der DE-A 10101695 bzw. WO 02/062737.

Weiterhin sind das Beispiel 1 aus der DE-A 10046957 (Stöchiometrie: $[Bi_2W_2O_9 \times 2WO_3]_{0,5} \cdot [Mo_{12}Co_{5,6}Fe_{2,94}Si_{1,59}K_{0,08}O_x]_1$) als Hohlzylinder (Ring) vollkatalysator der Geometrie 5 mm x 3 mm x 2 mm bzw. 5 mm x 2 mm x 2 mm (jeweils Außendurchmesser x Länge x Innendurchmesser), sowie die Schalenkatalysatoren 1, 2 und 3 aus der DE-A 10063162 (Stöchiometrie: $Mo_{12}Bi_{1,0}Fe_3Co_7Si_{1,6}K_{0,08}$), jedoch als ringförmige Schalenkatalysatoren entsprechender Schalendicke und auf Trägerringe der Geometrie 5 mm x 3 mm x 1,5 mm bzw. 7 mm x 3 mm x 1,5 mm (jeweils Außendurchmesser x Länge x Innendurchmesser) aufgebracht, geeignet.

Eine Vielzahl der für die Katalysatoren der Festbettkatalysatorschüttung geeigneten Multimetalloxidaktivmassen lässt sich unter der allgemeinen Formel I

$$Mo_{12}Bi_aFe_bX^1_cX^2_dX^3X^4_fO_n \qquad (I),$$

in der die Variablen nachfolgende Bedeutung aufweisen:

$X^1$ = Nickel und/oder Kobalt,
$X^2$ = Thallium, ein Alkalimetall und/oder ein Erdalkalimetall,
$X^3$ = Zink, Phosphor, Arsen, Bor, Antimon, Zinn, Cer, Blei und/oder Wolfram,
$X^4$ = Silicium, Aluminium, Titan und/oder Zirkonium,

a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,

subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel I in einfacher Weise dadurch hergestellt werden, dass

man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, Ammonium-Salze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Üblicherweise werden die Multimetalloxidaktivmassen der allgemeinen Formel I in der Festbettkatalysatorschüttung nicht in Pulverform sondern zu bestimmten Katalysatorgeometrien geformt eingesetzt, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren in der ersten Reaktionsstufe unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven O-xidmassen wird selbstredend an die gewünschte Schalendicke angepasst (vgl. EP-A 714 700).

Für die Katalysatoren der Reaktionsstufe geeignete Multimetalloxidaktivmassen sind ferner Massen der allgemeinen Formel II

$$[Y^1_{a'}Y^2_{b'}O_{x'}]_p[Y^3_{c'}Y^4_{d'}Y^5_{e'}Y^7_{f'}Y^7_{g'}Y^2_{h'}O_{y'}]_q \qquad (II),$$

in der die Variablen folgende Bedeutung haben:

$Y^1$ = nur Wismut oder Wismut und wenigstens eines der Elemente Tellur, Antimon, Zinn und Kupfer,
$Y^2$ = Molybdän oder Molybdän und Wolfram,
$Y^3$ = ein Alkalimetall, Thallium und/oder Samarium,
$Y^4$ = ein Erdalkalimetall, Nickel, Kobalt, Kupfer, Mangan, Zink, Zinn, Cadmium und/oder Quecksilber,
$Y^5$ = Eisen oder Eisen und wenigstens eines der Elemente Chrom und Cer,
$Y^6$ = Phosphor, Arsen, Bor und/oder Antimon,
$Y^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' = 8 bis 16,
x',y'= Zahlen, die durch die Wertigkeit und Häufigkeit der von Sauerstoff verschiedenen Elemente in II bestimmt werden und
p,q = Zahlen, deren Verhältnis p/q 0,1 bis 10 beträgt,

enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte erfindungsgemäße Multimetalloxidmassen II sind solche, in denen $Y^1$ nur Wismut ist.

Unter diesen werden wiederum jene bevorzugt, die der allgemeinen Formel III

$$[Bi_{a''}Z^2_{b''}O_{x''}]_{p''}\ [Z^2_{12}Z^3_{c''}Z^4_{d''}Fe_{e''}Z^5_{f''}Z^6_{g''}Z^7_{h''}O_{y''}]_{q''} \qquad (III)$$

in der die Varianten folgende Bedeutung haben:

$Z^2$ = Molybdän oder Molybdän und Wolfram,
$Z^3$ = Nickel und/oder Kobalt,
$Z^4$ = Thallium, ein Alkalimetall und/oder ein Erdalkalimetall,
$Z^5$ = Phosphor, Arsen, Bor, Antimon, Zinn, Cer und/oder Blei,
$Z^6$ = Silicium, Aluminium, Titan und/oder Zirkonium,
$Z^7$ = Kupfer, Silber und/oder Gold,

a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,
x",y"= Zahlen, die durch die Wertigkeit und Häufigkeit der von Sauerstoff verschiedenen Element in III bestimmt werden,
p",q"=Zahlen, deren Verhältnis p"/q" 0,1 bis 5, vorzugsweise 0,5 bis 2 beträgt,

entsprechen, wobei diejenigen Massen III ganz besonders bevorzugt werden, in denen $Z^2_{b''}$ = (Wolfram)$_{b''}$ und $Z^2_{12}$ =

$(Molybdän)_{12}$ ist.

Ferner ist es von Vorteil, wenn wenigstens 25 mol-% (bevorzugt wenigstens 50 mol-% und besonders bevorzugt wenigstens 100 mol-%) des gesamten Anteils $[Y^1_{a'}Y^2_{b'}O_{x'}]_p$ ($[Bi_{a''}Z^2_{b''}O_{x''}]_{p''}$) der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}$ $[Bi_{a''}Z^2_{b''}O_{x''}]$ vorliegen, deren Größtdurchmesser im Bereich 1 nm bis 100 μm liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z.B. in der EP-A 575897 sowie in der DE-A 19855913 beschrieben.

Erfindungsgemäß wesentlich ist, dass die Festbettkatalysatorschüttung aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt.

Die volumenspezifische (d.h., die auf die Einheit des jeweiligen Schüttungsvolumens normierte) Aktivität einer Festbettkatalysatorschüttungszone kann nun in über die Festbettkatalysatorschüttungszone im wesentlichen konstanter Weises dadurch eingestellt werden, dass man von einer Grundmenge einheitlich hergestellter Katalysatorformkörper ausgeht (ihre Schüttung entspricht der maximal erzielbaren volumenspezifischen Aktivität) und diese in der jeweiligen Festbettkatalysatorschüttungszone mit sich bezüglich der heterogen katalysierten partiellen Gasphasenoxidation im wesentlichen inert verhaltenden Formkörpern (Verdünnungsformkörper) homogen verdünnt. Je höher der Anteil der Verdünnungsformkörper gewählt wird, desto geringer ist die in einem bestimmten Volumen der Schüttung enthaltene Aktivmasse bzw. Katalysatoraktivität. Als Materialien für solche inerten Verdünnungsformkörper kommen prinzipiell alle diejenigen in Betracht, die sich auch als Trägermaterial für erfindungsgemäß geeignete Schalenkatalysatoren eignen.

Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungsformkörper solche wählen, deren Geometrie derjenigen der mit ihnen zu verdünnenden Katalysatorformkörper entspricht.

Erfindungsgemäß günstig ist es, wenn sich die chemische Zusammensetzung der verwendeten Aktivmasse über die gesamte Festbettkatalysatorschüttung nicht verändert. D.h., die für einen einzelnen Katalysatorformkörper verwendete Aktivmasse kann zwar ein Gemisch aus verschiedenen, die Elemente Mo, Fe und Bi enthaltenden, Multimetalloxiden sein, für alle Katalysatorformkörper der Festbettkatalysatorschüttung ist dann jedoch das gleiche Gemisch zu verwenden.

Eine in Strömungsrichtung des Reaktionsgasgemisches über die Festbettkatalysatorschüttung zonenweise zunehmende volumenspezifische Aktivität lässt sich für das erfindungsgemäße Verfahren somit in einfacher Weise z.B. dadurch einstellen, dass man die Schüttung in einer ersten Festbettkatalysatorschüttungszone mit einem hohen Anteil an inerten Verdünnungsformkörpem bezogen auf eine Sorte von Katalysatorformkörpern beginnt, und dann diesen Anteil an Verdünnungsformkörpem in Strömungsrichtung zonenweise verringert.

Eine zonenweise Zunahme der volumenspezifischen Aktivität ist aber auch z.B. dadurch möglich, dass man bei gleich bleibender Geometrie und Aktivmassenart eines Schalenkatalysatorformkörpers zonenweise die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse zonenweise den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich z.B. auch dadurch erzielen, dass man bei Mischungen aus Vollkatalysatoren und aus Schalenkatalysatoren (bei identischer Aktivmasse) in entsprechender Weise das Mischungsverhältnis verändert. Des weiteren lässt sich eine Variation der volumenspezifischen Aktivität durch den Einsatz von Katalysatorgeometrien mit unterschiedlicher Schüttdichte erzielen (z.B. bei Vollkatalysatoren mit identischer Aktivmassenzusammensetzung der verschiedenen Geometrien). Selbstredend lassen sich die beschriebenen Varianten auch kombiniert anwenden.

Natürlich können für die Festbettkatalysatorschüttung aber auch Mischungen aus Katalysatoren mit chemisch unterschiedlicher Aktivmassenzusammensetzung und als Folge dieser verschiedenen Zusammensetzung unterschiedliche Aktivität verwendet werden. Diese Mischungen können wiederum zonenweise in ihrer Zusammensetzung variiert und/oder mit unterschiedlichen Mengen inerter Verdünnungsformkörper verdünnt werden.

Vorab und/oder im Anschluss an die Festbettkatalysatorschüttung können sich ausschließlich aus Inertmaterial (z.B. nur Verdünnungsformkörper) bestehende Schüttungen befinden (sie werden in dieser Schrift begrifflich nicht der Festbettkatalysatorschüttung zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4 - 5 mm auf. Die Temperaturzonen A und B können sich beim erfindungsgemäßen Verfahren auch auf die Inertschüttungen erstrecken. Erfindungsgemäß vorteilhaft erfassen sowohl die Temperaturzone A als auch die Temperaturzone B jeweils nicht mehr als drei Festbettkatalysatorschüttungszonen (erfindungsgemäß zwingend wird wenigstens eine Festbettkatalysatorschüttungszone von beiden Temperaturzonen erfasst).

Erfindungsgemäß besonders vorteilhaft umfasst die gesamte Festbettkatalysatorschüttung nicht mehr als fünf, zweckmäßig nicht mehr als vier bzw. drei Festbettkatalysatorschüttungszonen.

Beim Übergang von einer Festbettkatalysatorschüttungszone in eine andere Festbettkatalysatorschüttungszone (in Strömungsrichtung des Reaktionsgasgemisches) sollte (bei einheitlicher Aktivmasse über die gesamte Festbettkatalysatorschüttung) die volumenspezifische Aktivmasse (d.h., das Gewicht der im Einheitsschüttungsvolumen enthaltenen Multimetalloxidmasse) erfindungsgemäß zweckmäßig um wenigstens 5 Gew.-%, bevorzugt um wenigstens 10 Gew.-% zunehmen (dies gilt insbesondere auch bei einheitlichen Katalysatorformkörpern über die gesamte Festbettkatalysatorschüttung). In der Regel wird diese Zunahme beim erfindungsgemäßen Verfahren nicht mehr als 50 Gew.-%, meist nicht mehr als 40 Gew.-% betragen. Ferner sollte bei einheitlicher Aktivmasse über die gesamte Festbettkatalysatorschüttung der Unterschied in der volumenspezifischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität erfindungsgemäß vorteilhaft nicht mehr als 50 Gew.-%, vorzugsweise nicht mehr als 40 Gew.-% und in der Regel nicht mehr als 30 Gew.-% betragen.

Häufig wird beim erfindungsgemäßen Verfahren die Festbettkatalysatorschüttung aus nur zwei Festbettkatalysatorschüttungszonen bestehen.

Erfindungsgemäß bevorzugt ist die in Strömungsrichtung des Reaktionsgasgemisches letzte Festbettkatalysatorschüttungszone unverdünnt. D.h., sie besteht vorzugsweise ausschließlich aus Katalysatorformkörpern. Im Bedarfsfall kann sie auch aus einer Schüttung aus Katalysatorformkörpern bestehen, deren volumenspezifische Aktivität z.B. durch Verdünnung mit Inertmaterial abgesenkt, z.B. um 10 %, ist.

Besteht die Festbettkatalysatorschüttung nur aus zwei Festbettkatalysatorschüttungszonen, ist es erfindungsgemäß in der Regel vorteilhaft (wie ganz allgemein beim erfindungsgemäßen Verfahren), wenn die Festbettkatalysatorschüttungszone mit der höchsten volumenspezifischen Aktivität nicht bis in die Temperaturzone A hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt). D.h., in günstiger Weise wird die Festbettkatalysatorschüttungszone mit der geringeren volumenspezifischen Aktivität in die Temperaturzone B hineinragen und die Festbettkatalysatorschüttungszone mit der höheren volumenspezifischen Aktivität in der Temperaturzone B beginnen und enden (d.h., hinter dem Übergang von der Temperaturzone A in die Temperaturzone B ihren Anfang haben).

Besteht die Festbettkatalysatorschüttung nur aus drei Festbettkatalysatorschüttungszonen, ist es erfindungsgemäß in der Regel vorteilhaft, wenn die Festbettkatalysatorschüttungszone mit der höchsten volumenspezifischen Aktivität nicht bis in die Temperaturzone A hineinragt sondern in der Temperaturzone B beginnt und endet, d.h., hinter dem Übergang von der Temperaturzone A in die Temperaturzone B ihren Anfang hat (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt). D.h., normalerweise wird in diesem Fall die Festbettkatalysatorschüttungszone mit der zweithöchsten volumenspezifischen Aktivität sowohl in die Temperaturzone A als auch in die Temperaturzone B hineinragen.

Besteht die Festbettkatalysatorschüttung aus vier Festbettkatalysatorschüttungszonen, ist es erfindungsgemäß in der Regel vorteilhaft, wenn die Festbettkatalysatorschüttungszone mit der dritthöchsten volumenspezifischen Aktivität sowohl in die Temperaturzone A als auch in die Temperaturzone B hineinragt (insbesondere dann, wenn in der Temperaturzone A und in der Temperaturzone B die Temperierung mittels eines fließenden Wärmeträgers erfolgt, der jeweils im Gegenstrom zum Reaktionsgasgemisch strömt).

Im Fall einer Gleichstromführung von Reaktionsgasgemisch und Wärmeträgern in den Temperaturzonen A und B kann es vorteilhaft sein, wenn beim erfindungsgemäßen Verfahren die Festbettkatalysatorschüttungszone mit

der höchsten volumenspezifischen Aktivität in die Temperaturzone A hineinragt.

Generell lässt sich die volumenspezifische Aktivität zwischen zwei Festbettkatalysatorschüttungszonen experimentell in einfacher Weise so differenzieren, dass unter identischen Randbedingungen (vorzugsweise den Bedingungen des ins Auge gefassten Verfahrens) über Festbettkatalysatorschüttungen derselben Länge, aber jeweils der Zusammensetzung der jeweiligen Festbettkatalysatorschüttungszone entsprechend, das gleiche Propen enthaltende Reaktionsgasausgangsgemisch geführt wird. Die höhere umgesetzte Menge an Propen weist die höhere volumenspezifische Aktivität aus.

Beträgt die Gesamtlänge der Festbettkatalysatorschüttung L, ist es erfindungsgemäß vorteilhaft, wenn sich im Bereich $X \pm L \cdot \frac{4}{100}$ bzw. im Bereich $X \pm L \cdot \frac{3}{100}$ bzw. im Be-100 100 reich $X \pm L \cdot \frac{2}{100}$ kein Übergang von einer Festbettkatalysatorschüttungszone in eine andere Festbettkatalysatorschüttungszone befindet, wobei X der Ort (die Stelle) innerhalb der Festbettkatalysatorschüttung ist, an dem der Übergang von der Temperaturzone A in die Temperaturzone B erfolgt.

Erfindungsgemäß bevorzugt ist beim erfindungsgemäßen Verfahren die Festbettkatalysatorschüttung in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h. z.B. auf einer Länge von 0,70 bis 1,50 m, bevorzugt 0,90 bis 1,20 m), jeweils der Gesamtlänge der Festbettkatalysatorschüttung 1, ein homogenes Gemisch aus Katalysatorformkörpern und Verdünnungsformkörpem (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.-%, oder 20 bis 40 Gew.-%, oder 25 bis 35 Gew.-% beträgt. Im Anschluss an diese erste Zone der Festbettkatalysatorschüttung befindet sich dann erfindungsgemäß vorteilhaft bis zum Ende der Länge der Festbettkatalysatorschüttung (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind. Das Vorgenannte trifft insbesondere dann zu, wenn in der Festbettkatalysatorschüttung als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

Das Vorgenannte trifft auch dann zu, wenn anstelle inerter Verdünnungsfórmkörper Schalenkatalysatorformkörper verwendet werden, deren Aktivmassenanteil um 2 bis 15 Gew.-% Punkte tiefer liegt, als der Aktivmassenanteil der am Ende der Festbettkatalysatorschüttung gegebenenfalls verwendeten Schalenkatalysatorformkörper.

Eine reine Inertmaterialschüttung, deren Länge, bezogen auf die Länge der Festbettkatalysatorschüttung, zweckmäßig 5 bis 20 % beträgt, leitet in Strömungsrichtung des Reaktionsgasgemisches in der Regel die Festbettkatalysatorschüttung ein. Sie wird normalerweise als Aufheizzone für das Reaktionsgasgemisch genutzt.

Erfindungsgemäß vorteilhaft erstreckt sich nun bei den vorgenannten Festbettkatalysatorschüttungen die Festbettkatalysatorschüttungszone mit der geringeren volumenspezifischen Aktivität noch auf 5 bis 20 % , häufig auf 5 bis 15 % ihrer Länge in die Temperaturzone B.

Erfindungsgemäß zweckmäßig erstreckt sich die Temperaturzone A auch auf eine gegebenenfalls angewendete Vorschüttung aus Inertmaterial.

In anwendungstechnisch zweckmäßiger Weise erfolgt die Durchführung der erfindungsgemäßen Reaktionsstufe des erfindungsgemäßen Verfahrens in einem Zweizonenrohrbündelreaktor, wie er z.B. in den DE-A’s 19910508, 19948523, 19910506 und 19948241 beschrieben ist. Eine bevorzugte Variante eines erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903218 offenbarten Zweizonenrohrbündelreaktoren sind für eine Durchführung der ersten Reaktionsstufe des erfindungsgemäßen Verfahrens geeignet.

D.h., in einfachster Weise befindet sich die erfindungsgemäß zu verwendende Festbettkatalysatorschüttung (eventuell mit vor- und/oder nachangeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Temperaturzone. D.h., in einfachster Weise umströmt z.B. ein Salzbad A denjenigen Abschnitt der Rohre (die Temperaturzone A), in welchem sich die oxidative Umsetzung des Propens (beim einfachen Durchgang) bis zum Erreichen eines Umsatzes im Bereich von 40 bis 80 mol-% vollzieht und ein Salzbad B umströmt den Abschnitt der Rohre (die Temperaturzone B), in welchem sich die oxidative Anschlussumsetzung des Propens (beim einfachen Durchgang) bis zum Erreichen eines Umsatzwertes von wenigstens 90 mol-% vollzieht (bei Bedarf können sich an die erfindungsgemäß anzuwendenden Temperaturzonen A,B weitere Temperaturzonen anschließen, die auf individuellen Temperaturen gehalten werden).

Anwendungstechnisch zweckmäßig umfasst die Reaktionsstufe des erfindungsgemäßen Verfahrens keine weiteren Temperaturzonen. D.h., das Salzbad B umströmt zweckmäßig den Abschnitt der Rohre, in welchem sich die oxidative Anschlussumsetzung des Propens (beim einfachen Durchgang) bis zu einem Umsatzwert ≥90 mol-%, oder ≥92 mol-% oder ≥94 mol-% oder mehr vollzieht.

Üblicherweise liegt der Beginn der Temperaturzone B hinter dem Heißpunktmaximum der Temperaturzone A.

Die beiden Salzbäder A,B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Temperaturzone A eine Gleichströmung und in der Temperaturzone B eine Gegenströmung (oder umgekehrt) angewandt werden.

Selbstverständlich kann man in allen vorgenannten Fallkonstellationen innerhalb der jeweiligen Temperaturzone der, relativ zu den Reaktionsrohren, erfolgenden Parallelströmung der Salzschmelze noch eine Querströmung überlagern, so dass die einzelne Temperaturzone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird beim erfindungsgemäßen Verfahren das Reaktionsgasausgangsgemisch der Festbettkatalysatorschüttung auf die Reaktionstemperatur vorerwärmt zugeführt.

Üblicherweise sind in den Zweizonenrohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m, bevorzugt 2,5 bis 3,5 m. In jeder Temperaturzone belegt die Festbettkatalysatorschüttung wenigstens 60 % bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. z.B. EP-B 468290).

Als Wärmeaustauschmittel eignen sich auch für die Zweizonenfahrweise insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren die Fließgeschwindigkeit innerhalb der beiden erforderlichen Wärmeaustauschmittelkreisläufen so gewählt, dass die Temperatur des Wärmeaustauschmittels von der Eintrittsstelle in die Temperaturzone bis zur Austrittstelle aus der Temperaturzone (bedingt durch die Exothermie der Reaktion) um 0 bis 15˚C ansteigt. D.h., das vorgenannte ΔT kann erfindungsgemäß 1 bis 10˚C, oder 2 bis 8˚C oder 3 bis 6˚C betragen.

Die Eintrittstemperatur des Wärmeaustauschmittels in die Temperaturzone A liegt erfindungsgemäß normalerweise im Bereich von 290 bis 380˚C, bevorzugt im Bereich von 305 bis 365˚C und besonders bevorzugt im Bereich von 310 bis 340˚C bzw. bis 330˚C. Bei Propenbelastungen der Festbettkatalysatorschüttung von ≥90 Nl/l•h und < 160 Nl/l•h wird die Eintrittstemperatur des Wärmeaustauschmittels in die Temperaturzone B erfindungsgemäß ebenfalls im Bereich von 290 bis 380˚C, gleichzeitig aber normalerweise erfindungsgemäß zweckmäßig ≥0˚C bis ≤20˚C oder ≤10˚C, bzw. ≥0˚C und ≤5˚C, oder häufig ≥0˚C und ≤3˚C unterhalb der Eintrittstemperatur des in die Temperaturzone A eintretenden Wärmeaustauschmittels liegen. Bei Propenbelastungen der Festbettkatalysatorschüttung von ≥160 Nl/l•h und (in der Regel) ≤300 Nl/l•h (bzw. 600 Nl/l•h) wird die Eintrittstemperatur des Wärmeaustauschmittels in die Temperaturzone B erfindungsgemäß ebenfalls im Bereich von 290 bis 380˚C, aber normalerweise erfindungsgemäß zweckmäßig > 0˚C und ≤50˚C, oder ≥5˚C und ≤45˚C, oder ≥10˚C und ≤40˚C, oder ≥15˚C und ≤30˚C oder ≤35˚C (z.B. 20˚C oder 25˚C) oberhalb der Eintrittstemperatur des in die Temperaturzone A eintretenden Wärmeaustauschmittels liegen.

Es sei an dieser Stelle auch noch einmal darauf hingewiesen, dass für eine Durchführung der Reaktionsstufe des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Temperaturzone B eine Teilmenge an die Temperaturzone A abzuführen, um gegebenenfalls ein Anwärmen eines kalten Reaktionsgasausgangsgemisches oder eines kalten Kreisgases zu bewirken. Ferner kann die Rohrbündelcharakteristik innerhalb einer individuellen Temperaturzone wie in der EP-A 382098 beschrieben gestaltet werden.

Aus dem die Reaktionsstufe verlassenden Produktgasgemisch kann das Acrolein in an sich bekannter Weise abgetrennt und das dabei verbleibende Restgas in an sich bekannter Weise wenigstens teilweise als Verdünnungsgas

in die Propenpartialoxidation rückgeführt werden. In zweckmäßiger Weise wird man es jedoch zur Beschickung einer nachfolgenden heterogen katalysierten Partialoxidation des darin enthaltenen Acroleins zu Acrylsäure verwenden. Zeckmäßigerweise wird man es vor dem Eintritt in eine solche nachfolgende Reaktionsstufe auf direkte und/oder indirekte Weise abkühlen, um so eine Nachvollverbrennung von Teilen des in der Propenoxidationsstufe gebildeten Acroleins zu unterdrücken.

Üblicherweise wird dazu zwischen die beiden Reaktionsstufen ein Nachkühler geschaltet. Dies kann im einfachsten Fall ein indirekter Rohrbündelwärmeträger sein. Anschließend wird meist noch Sauerstoff in Form von Luft ergänzt, um den für eine solche Acroleinoxidation vorteilhaften überstöchiometrischen Sauerstoffgehalt zur Verfügung zu stellen. Mit Vorteil wird eine solche Acroleinpartialoxidation in Analogie zum erfindungsgemäßen Verfahren durchgeführt.

Der Propenanteil im Reaktionsgasausgangsgemisch kann beim erfindungsgemäßen Verfahren z.B. bei Werten von 4 bis 15 Vol.%, häufig bei 5 bis 12 Vol.-% bzw. 5 bis 8 Vol.-% liegen (jeweils bezogen auf das Gesamtvolumen).

Häufig wird man das erfindungsgemäße Verfahren bei einem Propen : Sauerstoff : indifferente Gase (einschließlich Wasserdampf) Volumenverhältnis im Reaktionsgasausgangsgemisch 1 von 1 : (1,0 bis 3,0): (5 bis 25), vorzugsweise 1 : (1,7 bis 2,3): (10 bis 15) durchführen. Im Regelfall wird das indifferente (inerte) Gas zu wenigstens 20 % seines Volumens aus molekularem Stickstoff bestehen. Es kann aber auch zu ≥30 Vol.-%, oder zu ≥40 Vol.-%, oder zu ≥50 Vol.-%, oder zu ≥60 Vol.-%, oder zu ≥70 Vol.-%, oder zur ≥80 Vol.-%, oder zu ≥90 Vol.-%, oder zu ≥95 Vol.-% aus molekularem Stickstoff bestehen (generell sollen in dieser Schrift inerte Verdünnungsgase solche sein, die sich beim einmaligen Durchgang durch die Reaktionsstufe zu weniger als 5 %, bevorzugt zu weniger als 2 % umsetzen; das sind neben molekularem Stickstoff z.B. Gase wie Propan, Ethan, Methan, Pentan, Butan, $CO_2$, CO, Wasserdampf und/oder Edelgase). Natürlich kann das inerte Verdünnungsgas beim erfindungsgemäßen Verfahren auch zu bis zu 50 mol-%, bzw. bis zu 75 mol-% und mehr aus Propan bestehen. Bestandteil des Verdünnungsgases kann auch Kreisgas sein, wie es nach der Abtrennung des Acroleins und/oder der Acrylsäure (z.B. im Fall einer nachgeschalteten zweiten Oxidationsstufe) aus dem Produktgasgemisch verbleibt.

Erfindungsgemäß günstige Reaktionsgasausgangsgemische sind z.B. auch solche, die aus

| | |
|---|---|
| 6 bis 15 (bevorzugt 7 bis 11) Vol-% | Propen, |
| 4 bis 20 (bevorzugt 6 bis 12) Vol.-% | Wasser, |
| ≥0 bis 10 (bevorzugt ≥0 bis 5) Vol.-% | von Propen, Wasser Sauerstoff und Stick-stoff verschiedenen Bestandteilen, |

soviel molekularem Sauerstoff, dass das molare Verhältnis von enthaltenem molekularem Sauerstoff zu enthaltenem Propen 1,5 bis 2,5 (bevorzugt 1,6 bis 2,2) beträgt, und als Restmenge bis zu 100 Vol.-% Gesamtmenge aus molekularem Stickstoff zusammengesetzt sind,
wie sie die DE-A 10302715 empfiehlt.

An dieser Stelle sei noch festgehalten, dass als Aktivmassen für die Festbettkatalysatorschüttung auch die Multimetalloxidmassen der DE-A 10261186 günstig sind.

Erfindungsgemäß günstige Ausgestaltungen eines Zweizonenrohrbündelreaktors für die erfindungsgemäße Reaktionsstufe können wie folgt beschaffen sein (die konstruktive Detailgestaltung kann wie in den Gebrauchsmusteranmeldungen 202 19 277.6, 2002 19 278.4 und 202 19 279.2 bzw. in den PCT-Anmeldungen PCT/EP02/14187, PCT/EP02/14188 oder PCT/EP02/14189 erfolgen):

Kontaktrohre:

| | |
|---|---|
| Material der Kontaktrohre: | ferritischer Stahl; |
| Abmessungen der Kontaktrohre: | z.B. 3500 mm Länge; |
| | z.B. 30 mm Außendurchmesser; |
| | z.B. 2 mm Wandstärke; |

Anzahl der Kontaktrohre im Rohrbündel: z.B. 30000, oder 28000, oder 32000, oder 34000; zusätzlich bis zu 10 Thermorohre (wie in EP-A 873 783 und EP-A 12 70 065 beschrieben), die wie die Kontaktrohre beschickt sind (schneckenartig von ganz außen nach innen drehend) z.B. der selben Länge und Wandstärke, aber mit einem Außendurchmesser von z.B. 33,4 mm und einer zentrierten Thermohülse von z.B. 8 mm Außendurchmesser und z.B. 1 mm Wandstärke;

Reaktor (Material wie die Kontaktrohre):

Zylinderförmiger Behälter eines Innendurchmessers von 6000 - 8000 mm; Reaktorhauben plattiert mit Edelstahl des Typs 1,4541; Plattierungsdicke: einige mm; ringförmig angeordnetes Rohrbündel, z.B. mit einem freien zentralen Raum;

Durchmesser des zentralen freien Raumes: z.B. 1000 - 2500 mm (z.B. 1200 mm, oder 1400 mm, oder 1600 mm, oder 1800 mm, oder 2000 mm, oder 2200 mm, oder 2400 mm);
normalerweise homogene Kontaktrohrverteilung im Rohrbündel (6 äquidistante Nachbarrohre pro Kontaktrohr), Anordnung in gleichseitigem Dreieck, Kontaktrohrteilung (Abstand der zentrischen Innenachsen von zueinander nächstliegenden Kontaktrohren): 35 - 45 mm, z.B. 36 mm, oder 38 mm, oder 40 mm, oder 42 mm, oder 44 mm;
die Kontaktrohre sind mit ihren Enden in Kontaktrohrböden (oberer Boden und unterer Boden z.B. mit einer Dicke von 100 - 200 mm) abdichtend befestigt und münden am oberen Ende in eine mit dem Behälter verbundene Haube, die einen Zulaß für das Reaktionsgasausgangsgemisch aufweist; ein z.B. auf der halben Kontaktrohrlänge befindliches Trennblech einer Dicke von 20 - 100 mm, teilt den Reaktorraum symmetrisch in zwei Temperaturzonen A (obere Zone) und B (untere Zone); jede Temperaturzone wird durch eine Umlenkscheibe in 2 äquidistante Längsabschnitte geteilt;
die Umlenkscheibe weist bevorzugt Ringgeometrie auf; die Kontaktrohre sind mit Vorteil am Trennblech abdichtend befestigt; an den Umlenkscheiben sind sie nicht abdichtend befestigt, so dass die Querströmungsgeschwindigkeit der Salzschmelze innerhalb einer Zone möglichst konstant ist;
jede Zone wird durch eine eigene Salzpumpe mit Salzschmelze als Wärmeträger versorgt; die Zufuhr der Salzschmelze ist z.B. unterhalb der Umlenkscheibe und die Entnahme ist z.B. oberhalb der Umlenkscheibe;
aus beiden Salzschmelzekreisen wird z.B. ein Teilstrom entnommen und z.B. in einem gemeinsamen oder zwei getrennten indirekten Wärmetauschern abgekühlt (Dampferzeugung);
im ersten Fall wird der abgekühlte Salzschmelzestrom aufgeteilt, mit dem jeweiligen Reststrom vereinigt und durch die jeweilige Pumpe in den entsprechenden Ringkanal, der die Salzschmelze über den Behälterumfang verteilt, in den Reaktor gedrückt;
durch im Reaktormantel befindliche Fenster gelangt die Salzschmelze zum Rohrbündel; die Einströmung erfolgt z.B. in radialer Richtung zum Rohrbündel;
die Salzschmelze fließt in jeder Zone der Vorgabe des Umlenkblechs folgend z.B. in der Abfolge

- von außen nach innen,
- von innen nach außen,

um die Kontaktrohre;
durch um den Behälterumfang angebrachte Fenster sammelt sich die Salzschmelze an jedem Zonenende in einem um den Reaktormantel angebrachten Ringkanal, um einschließlich Teilstromkühlung im Kreis gepumpt zu werden;
über jede Temperaturzone wird die Salzschmelze von unten nach oben geführt;

Das Reaktionsgasgemisch verlässt den Reaktor der erfindungsgemäßen Reaktionsstufe mit einer Temperatur wenige Grad höher als die Salzbadeintrittstemperatur. Das Reaktionsgasgemisch wird für die Weiterverarbeitung in einer nachfolgenden Acroleinoxidationsstufe zweckmäßigerweise in einem separaten Nachkühler, der dem Reaktor der 1. Stufe nachgeschaltet ist, auf 220˚C bis 280˚C, bevorzugt 240˚C bis 260˚C abgekühlt.

Der Nachkühler ist in der Regel unterhalb des unteren Rohrbodens angeflanscht und besteht normalerweise aus Rohren mit ferritischem Stahl. In die Rohre des Nachkühlers sind mit Vorteil innen Edelstahl-Blechspiralen, die teil- oder vollgewendelt sein können, zur Verbesserung des Wärmeübergangs eingeführt.

Salzschmelze:

Als Salzschmelze kann ein Gemisch aus 53 Gew.-% Kaliumnitrat, 40 Gew.-% Natriumnitrit und 7 Gew.-% Natriumnitrat verwendet werden; beide Temperaturzonen und der Nachkühler wenden mit Vorteil eine Salzschmelze derselben Zusammensetzung an; die in den Temperaturzonen umgepumpte Salzmenge kann je Zone ca. 10000 $m^3$/h betragen.

Stromführung:

das Reaktionsgasausgangsgemisch strömt zweckmäßig von oben nach unten durch den Erststufenreaktor, während die unterschiedlich temperierten Salzschmelzen der einzelnen Zonen zweckmäßig von unten nach oben gefördert werden;

Kontaktrohr- und Thermorohrbeschickung (von oben nach unten) z.B.:

Abschnitt 1: 50 cm Länge Steatitringe der Geometrie 7 mm x 7 mm x 4 mm (Außendurchmesser x Länge x Innendurchmesser) als Vorschüttung.

Abschnitt 2: 140 cm Länge Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-

% Vollkatalysator aus Abschnitt 3.

Abschnitt 3: 160 cm Länge Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: $[Bi_2W_2O_9 \times 2\ WO_3]_{0,5}\ [Mo_{12}Co_{5,5}Fe_{2,94}Si_{1,59}K_{0,08}O_x]_1$).

In der vorgenannten Beschickung kann der Vollkatalysator aus Beispiel 1 der DE-A 10046957 auch ersetzt werden durch:

a) einen Katalysator gemäß Beispiel 1 c aus der EP-A 15565 oder einen gemäß diesem Beispiel herzustellenden Katalysator, der jedoch die Aktivmasse $Mo_{12}Ni_{6,5}Zn_2Fe_2Bi_1P_{0,0065}K_{0,06}O_x \cdot 10\ SiO_2$ aufweist;

b) Beispiel Nr. 3 aus der DE-A 19855913 als Hohlzylindervollkatalysator der Geometrie 5 mm × 3 mm × 2 mm bzw. 5 mm × 2 mm × 2 mm;

c) Multimetalloxid II - Vollkatalysator gemäß Beispie 1 der DE-A 19746210;

d) einen der Schalenkatalysatoren 1, 2 und 3 aus der DE-A 10063162, jedoch bei gleicher Schalendicke auf Trägerringe der Geometrie 5 mm x 3 mm x 1,5 mm bzw. 7 mm x 3 mm x 1,5 mm aufgebracht.

Im Übrigen wird die Festbettkatalysatorschüttung erfindungsgemäß zweckmäßig so gewählt (z.B. durch Verdünnung mit z.B. Inertmaterial), dass der Temperaturunterschied zwischen dem Heißpunktmaximum des Reaktionsgasgemisches in den einzelnen Temperaturzonen und der jeweiligen Temperatur der Temperaturzone in der Regel 80°C nicht überschreitet. Meist beträgt dieser Temperaturunterschied ≤70°C, häufig liegt er bei 20 bis 70°C, vorzugsweise ist dieser Temperaturunterschied gering. Außerdem wird die Festbettkatalysatorschüttung aus Sicherheitsgründen in dem Fachmann an sich bekannter Weise so gewählt (z.B. durch Verdünnung mit z.B. Inertmaterial), dass die "peak-to-salt-temperature sensitivity" gemäß Definition in der EP-A 1106598 ≤ 9°C, oder ≤7°C, oder ≤5°C, oder ≤3°C beträgt.

Beispiele

Ein Reaktionsrohr (V2A Stahl; 30 mm Außendurchmesser, 2 mm Wandstärke, 26 mm Innendurchmesser, Länge: 350 cm, sowie ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann) wird von oben nach unten wie folgt beschickt:

Abschnitt 1: 50 cm Länge Steatitringe der Geometrie 7 mm x 7 mm x 4 mm (Außendurchmesser x Länge x Innendurchmesser) als Vorschüttung.

Abschnitt 2: 140 cm Länge Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt 3.

Abschnitt 3: 160 cm Länge Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: $[Bi_2W_2O_9 \times 2WO_3]_{0,5}\ [Mo_{12}Co_{5,5}Fe_{2,94}Si_{1,59}K_{0.08}O_x]_1$).

Von oben nach unten werden die ersten 175 cm mittels eines im Gegenstrom gepumpten Salzbades A thermostatisiert. Die zweiten 175 cm werden mittels eines im Gegenstrom gepumpten Salzbades B thermostatisiert.

Die vorstehend beschriebene erste Reaktionsstufe wird mit einem Reaktionsgasausgangsgemisch 1 der nachfolgenden Zusammensetzung kontinuierlich beschickt, wobei die Belastung und die Thermostatisierung des ersten Reaktionsrohres variiert werden:

6 bis 6,5 Vol.-% Propen,
3 bis 3,5 Vol.-% $H_2O$,
0,3 bis 0,5 Vol.-% CO,
0,8 bis 1,2 Vol.-% $CO_2$,
0,01 bis 0,04 Vol.-% Acrolein,
10,4 bis 10,7 Vol.-% $O_2$ und

als Restmenge ad 100 % molekularer Stickstoff.

Das Reaktionsgasgemisch durchströmt das Reaktionsrohr von oben nach unten.

Der Druck am Eingang des Reaktionsrohres variiert in Abhängigkeit von der gewählten Propenbelastung zwischen 2,3 und 3,1 bar.

Dem Produktgasgemisch wird am Reaktionsrohrausgang eine kleine Probe für die gaschromatographische Analyse genommen. Am Ende der Temperaturzone A befindet sich ebenfalls eine Analysenstelle.

Die Ergebnisse in Abhängigkeit von Belastungen und Salzbadtemperaturen zeigt die nachfolgende Tabelle 1 (der Buchstabe B in Klammern bedeutet Beispiel).

$T_A$, $T_B$, stehen für die Temperaturen der umgepumpten Salzbäder in den Temperaturzonen A und B.

$U_A^P$ ist der Propenumsatz am Ende der Temperaturzone A in mol-%.

$U_B^P$ ist der Propenumsatz am Ende der Temperaturzone B in mol-%.

$S^{AC}$ ist die Selektivität der Acroleinbildung im Produktgasgemisch bezogen auf umgesetztes Propen in mol-%.

$S^{AA}$ ist die Selektivität der Acrylsäurenebenproduktbildung im Produktgasgemisch bezogen auf umgesetztes Propen in mol-%.

$T^{maxA}$, $T^{maxB}$ stehen für die höchste Temperatur des Reaktionsgasgemisches innerhalb der Temperaturzonen A und B in ˚C.

Tabelle 1

| Propenbelastung (Nl/l•h) | $T_A$ | $T_B$ | $T^{maxA}$ | $T^{maxB}$ | $U_A^P$ | $U_B^P$ | $S^{AC}$ | $S^{AA}$ |
|---|---|---|---|---|---|---|---|---|
| 130 (B) | 319 | 319 | 384 | 351 | 66,7 | 95,1 | 92,6 | 5,1 |
| 130 (B) | 327 | 313 | 400 | 330 | 70,3 | 94,8 | 93,9 | 4,4 |
| 185 (B) | 322 | 336 | 380 | 368 | 64,5 | 94,9 | 90,6 | 7,4 |

Vergleichsbeispiele

Alles wird wie in den Beispielen durchgeführt. Die Längen der Beschickungsabschnitte der Festbettkatalysatorbeschickung werden jedoch wie folgt geändert:

Abschnitt 2: 125 cm anstelle 140 cm.
Abschnitt 3: 175 cm anstelle 160 cm.

Die Ergebnisse zeigt die Tabelle 2. (V) bedeutet Vergleichsbeispiel.

Tabelle 2

| Propenbelastung (Nl/l•h) | $T_A$ | $T_B$ | $T^{maxA}$ | $T^{maxB}$ | $U_A^P$ | $U_B^P$ | $S^{AC}$ | $S^{AA}$ |
|---|---|---|---|---|---|---|---|---|
| 130 (V) | 319 | 315 | 384 | 360 | 66,4 | 94,9 | 91,8 | 5,6 |
| 130 (V) | 327 | 310 | 400 | 339 | 70,2 | 95,0 | 93,2 | 4,8 |
| 185 (V) | 322 | 331 | 380 | 377 | 64,7 | 94,8 | 89,9 | 7,8 |

Im Vergleich mit den Ergebnissen in Tabelle 1 werden bei vergleichbaren $U_B^P$ Werten geringere $S^{AC}$ erzielt.

**Patentansprüche**

1. Verfahren der heterogen katalysierten partiellen Gasphasenoxidation von Propen zu Acrolein bei dem man ein Propen, molekularen Sauerstoff und wenigstens ein Inertgas enthaltendes Reaktionsgasausgangsgemisch, das den molekularen Sauerstoff und das Propen in einem molaren Verhältnis $O_2$:$C_3H_6$ ≥1 enthält, in einer Reaktionsstufe

mit der Maßgabe über eine Festbettkatalysatorschüttung, deren Aktivmasse wenigstens ein die Elemente Mo, Fe und Bi enthaltendes Multimetalloxid ist, führt, dass

- die Festbettkatalysatorschüttung in zwei räumlich aufeinanderfolgenden Temperaturzonen A, B angeordnet ist,
- sowohl die Temperatur der Temperaturzone A als auch die Temperatur der Temperaturzone B eine Temperatur im Bereich von 290 bis 380°C ist,
- die Festbettkatalysatorschüttung aus wenigstens zwei räumlich aufeinander folgenden Festbettkatalysatorschüttungszonen besteht, wobei die volumenspezifische Aktivität innerhalb einer Festbettkatalysatorschüttungszone im wesentlichen konstant ist und in Strömungsrichtung des Reaktionsgasgemisches beim Übergang von einer Festbettkatalysatorschüttungszone in eine andere Festbettkatalysatorschüttungszone sprunghaft zunimmt,
- sich die Temperaturzone A bis zu einem Umsatz des Propens von 40 bis 80 mol.-% erstreckt,
- bei einmaligem Durchgang des Reaktionsgasausgangsgemisches durch die gesamte Festbettkatalysatorschüttung der Propenumsatz ≥90 mol.-% und die Selektivität der Acroleinbildung, bezogen auf umgesetztes Propen, ≥90 mol.-% beträgt,
- die zeitliche Abfolge, in der das Reaktionsgasgemisch die Temperaturzonen A, B durchströmt, der alphabetischen Abfolge der Temperaturzonen entspricht,
- die Belastung der Festbettkatalysatorschüttung mit dem im Reaktionsgasausgangsgemisch enthaltenen Propen ≥90 Nl Propen/l Festbettkatalysatorschüttung • h beträgt, und
- die Differenz $T^{maxA}$ - $T^{maxB}$, gebildet aus der höchsten Temperatur $T^{maxA}$, die das Reaktionsgasgemisch innerhalb der Temperaturzone A aufweist, und der höchsten Temperatur $T^{maxB}$, die das Reaktionsgasgemisch innerhalb der Temperaturzone B aufweist, ≥0°C beträgt,

**dadurch gekennzeichnet ist, dass** der Übergang von der Temperaturzone A in die Temperaturzone B in der Festbettkatalysatorschüttung nicht mit einem Übergang von einer Festbettkatalysatorschüttungszone in eine andere Festbettkatalysatorschüttungszone zusammenfällt.

2. Verfahren nach Anspruch 1, **dadurch gekennzeichnet, dass** $T^{maxA}$-$T^{maxB}$ ≥3°C und ≤70°C beträgt.

3. Verfahren nach Anspruch 1, **dadurch gekennzeichnet, dass** $T^{maxA}$-$T^{maxB}$ ≥20°C und ≤60°C beträgt.

4. Verfahren nach Anspruch 1 bis 3, **dadurch gekennzeichnet, dass** die Propenbelastung der Festbettkatalysatorschüttung ≥90 Nl/l • h und < 160 Nl/l • h beträgt.

5. Verfahren nach einem der Ansprüche 1 bis 3, **dadurch gekennzeichnet, dass** die Propenbelastung der Festbettkatalysatorschüttung ≥160 Nl/l • h und ≤300 Nl/l • h beträgt.

6. Verfahren nach einem der Ansprüche 1 bis 5, **dadurch gekennzeichnet, dass** die Temperatur der Reaktionszone A 305 bis 365°C beträgt.

7. Verfahren nach einem der Ansprüche 1 bis 5, **dadurch gekennzeichnet, dass** die Temperatur der Reaktionszone A 310 bis 340°C beträgt.

8. Verfahren nach einem der Ansprüche 1 bis 7, **dadurch gekennzeichnet, dass** sich die Temperaturzone A bis zu einem Umsatz des Propens von 50 bis 70 mol.-% erstreckt.

9. Verfahren nach einem der Ansprüche 1 bis 8, **dadurch gekennzeichnet, dass** sich die Temperaturzone A bis zu einem Umsatz des Propens von 60 bis 70 mol.-% erstreckt.

10. Verfahren nach einem der Ansprüche 1 bis 9, **dadurch gekennzeichnet, dass** das Verhältnis $O_2$:Propen im Reaktionsgasausgangsgemisch 1 bis 2 beträgt.

11. Verfahren nach einem der Ansprüche 1 bis 10, **dadurch gekennzeichnet, dass** die chemische Zusammensetzung der verwendeten Aktivmasse über die gesamte Festbettkatalysatorschüttung unverändert ist.

12. Verfahren nach einem der Ansprüche 1 bis 11, **dadurch gekennzeichnet, dass** die gesamte Festbettkatalysatorschüttung nicht mehr als vier Festbettkatalysatorschüttungszonen umfasst.

**13.** Verfahren nach einem der Ansprüche 1 bis 12, **dadurch gekennzeichnet, dass** bei einheitlicher Aktivmasse über die gesamte Festbettkatalysatorschüttung beim Übergang von einer Festbettkatalysatorschüttungszone in eine andere Festbettkatalysatorschüttungszone in Strömungsrichtung des Reaktionsgasgemisches die volumenspezifische Aktivmasse um wenigstens 5 Gew.-% zunimmt.

**14.** Verfahren nach einem der Ansprüche 1 bis 12, **dadurch gekennzeichnet, dass** bei einheitlicher Aktivmasse über die gesamte Festbettkatalysatorschüttung beim Übergang von einer Festbettkatalysatorschüttungszone in eine andere Festbettkatalysatorschüttungszone in Strömungsrichtung des Reaktionsgasgemisches die volumenspezifische Aktivmasse um wenigstens 10 Gew.-% zunimmt.

**15.** Verfahren nach einem der Ansprüche 1 bis 14, **dadurch gekennzeichnet, dass** bei einheitlicher Aktivmasse über die gesamte Festbettkatalysatorschüttung der Unterschied in der volumenspezifischen Aktivmasse derjenigen Festbettkatalysatorschüttungszone mit der geringsten volumenspezifischen Aktivität und derjenigen Festbettkatalysatorschüttungszone mit der höchsten volumenspezifischen Aktivität nicht mehr als 40 Gew.-% beträgt.

**16.** Verfahren nach einem der Ansprüche 1 bis 15, **dadurch gekennzeichnet, dass** die in Strömungsrichtung des Reaktionsgasgemisches letzte Festbettkatalysatorschüttungszone unverdünnt ist und nur aus Katalysatorformkörpern besteht.

**17.** Verfahren nach einem der Ansprüche 1 bis 16, **dadurch gekennzeichnet, dass** die Festbettkatalysatorschüttungszone mit der höchsten volumenspezifischen Aktivität nicht bis in die Temperaturzone A hineinragt.

**18.** Verfahren nach einem der Ansprüche 1 bis 17, **dadurch gekennzeichnet, dass** sich in der Festbettkatalysatorschüttung im Bereich $X \pm L \cdot \frac{4}{100}$ kein Übergang von einer Festbettkatalysatorschüttungszone in eine andere Festbettkatalysatorschüttungszone befindet, wobei L die Länge der Festbettkatalysatorschüttung und X die Stelle innerhalb der Festbettkatalysatorschüttung ist, wo der Übergang von der Temperaturzone A in die Temperaturzone B erfolgt.

## Claims

**1.** A process for partially oxidizing propene to acrolein in the gas phase under heterogeneous catalysis by conducting a starting reaction gas mixture comprising propene, molecular oxygen and at least one inert gas, and containing the molecular oxygen and the propene in a molar $O_2 : C_3H_6$ ratio of ≥1, in a reaction stage over a fixed catalyst bed whose active composition is at least one multimetal oxide comprising the elements Mo, Fe and Bi, with the proviso that

- the fixed catalyst bed is arranged in two spatially successive temperature zones A, B,
- both the temperature of temperature zone A and the temperature of temperature zone B is a temperature in the range from 290 to 380°C,
- the fixed catalyst bed consists of at least two spatially successive fixed catalyst bed zones, and the volume-specific activity within one fixed catalyst bed zone is substantially constant and increases sharply in the flow direction of the reaction gas mixture at the transition from one fixed catalyst bed zone to another fixed catalyst bed zone,
- the temperature zone A extends up to a conversion of the propene of from 40 to 80 mol%,
- on single pass of the starting reaction gas mixture through the entire fixed catalyst bed, the propene conversion is ≥ 90 mol% and the selectivity of acrolein formation based on converted propene is ≥ 90 mol%,

the sequence in time in which the reaction gas mixture flows through the temperature zones A, B corresponds to the alphabetic sequence of the temperature zones,
- the hourly space velocity of the propene contained in the starting reaction gas mixture on the fixed catalyst bed is ≥ 90 1 (STP) of propene/l of fixed bed catalyst·h, and
- the difference $T^{maxA} - T^{maxB}$, formed from the highest temperature $T^{maxA}$ which the reaction gas mixture has within temperature zone A, and the highest temperature $T^{maxB}$ which the reaction gas mixture has within temperature zone B is ≥ 0°C,

wherein the transition from temperature zone A to temperature zone B in the fixed catalyst bed does not coincide with a transition from one fixed catalyst bed zone to another fixed catalyst bed zone.

**2.** The process according to claim 1, wherein $T^{maxA}$ - $T^{maxB}$ is ≥ 3˚C and ≤ 70˚C.

**3.** The process according to claim 1, wherein $T^{maxA}$ - $T^{maxB}$ is ≥ 20˚C and ≤ 60˚C.

**4.** The process according to any of claims 1 to 3, wherein the propene hourly space velocity on the fixed catalyst bed is ≥ 90 1 (STP)/l⊙h and < 160 1 (STP)/l⊙h.

**5.** The process according to any of claims 1 to 3, wherein the propene hourly space velocity on the fixed catalyst bed is ≥ 160 1 (STP)/l⊙h and ≤ 300 1 (STP)/l⊙h.

**6.** The process according to any of claims 1 to 5, wherein the temperature of reaction zone A is from 305 to 365˚C.

**7.** The process according to any of claims 1 to 5, wherein the temperature of reaction zone A is from 310 to 340˚C.

**8.** The process according to any of claims 1 to 7, wherein temperature zone A extends to a propene conversion of from 50 to 70 mol%.

**9.** The process according to any of claims 1 to 8, wherein temperature zone A extends to a propene conversion of from 60 to 70 mol%.

**10.** The process according to any of claims 1 to 9, wherein the $O_2$:propene ratio in the starting reaction gas mixture is from 1 to 2.

**11.** The process according to any of claims 1 to 10, wherein the chemical composition of the active composition used is unchanged over the entire fixed catalyst bed.

**12.** The process according to any of claims 1 to 11, wherein the entire fixed catalyst bed comprises not more than 4 fixed catalyst bed zones.

**13.** The process according to any of claims 1 to 12, wherein, when the active composition is uniform over the entire fixed catalyst bed, the volume-specific active composition in the flow direction of the reaction gas mixture increases by at least 5% by weight at the transition from one fixed catalyst bed zone to another fixed catalyst bed zone.

**14.** The process according to any of claims 1 to 12, wherein, when the active composition is uniform over the entire fixed catalyst bed, the volume-specific active composition in the flow direction of the reaction gas mixture increases by at least 10% by weight at the transition from one fixed catalyst bed zone to another fixed catalyst bed zone.

**15.** The process according to any of claims 1 to 14, wherein, when the active composition is uniform over the entire fixed catalyst bed, the difference in the volume-specific active composition between the fixed catalyst bed zone having the lowest volume-specific activity and the fixed catalyst bed zone having the highest volume-specific activity is not more than 40% by weight.

**16.** The process according to any of claims 1 to 15, wherein the last fixed catalyst bed zone in the flow direction of the reaction gas mixture is undiluted and consists only of shaped catalyst bodies.

**17.** The process according to any of claims 1 to 16, wherein the fixed catalyst bed zone having the highest volume-specific activity does not extend into temperature zone A.

**18.** The process according to any of claims 1 to 17, wherein there is no transition from one fixed catalyst bed zone to another fixed catalyst bed zone in the fixed catalyst bed within the range of $X \pm L \cdot \frac{4}{100}$ where L is the length of the fixed catalyst bed and X is the point within the fixed catalyst bed of transition from temperature zone A to temperature zone B.

## Revendications

1. Procédé d'oxydation partielle en phase gazeuse, catalysée par voie hétérogène de propène en acroléine, dans lequel on guide un mélange réactionnel gazeux initial contenant du propène, de l'oxygène moléculaire et au moins un gaz inerte, qui contient l'oxygène moléculaire et le propène dans un rapport molaire $O_2:C_3H_6 \geq 1$, dans une étape de réaction sur un catalyseur en lit fixe en vrac, dont la masse active est au moins un oxyde multimétallique contenant des éléments Mo, Fe et Bi, à condition

   - que le catalyseur en lit fixe en vrac soit disposé dans deux zones de température consécutives dans l'espace A, B,
   - que tant la température de la zone de température A que la température de la zone de température B soit une température dans la plage de 290 à 380°C,
   - que le catalyseur en lit fixe en vrac soit constitué par au moins deux zones de catalyseur en lit fixe en vrac successives dans l'espace, l'activité spécifique volumique dans une zone de catalyseur en lit fixe en vrac étant sensiblement constante et augmentant par bonds dans le sens de l'écoulement du mélange réactionnel gazeux lors de la transition d'une zone de catalyseur en lit fixe en vrac dans une autre zone de catalyseur en lit fixe en vrac,
   - que la zone de température A s'étende jusqu'à une conversion du propène de 40 à 80% en mole,
   - que lors du passage unique du mélange réactionnel gazeux initial à travers la totalité du catalyseur en lit fixe en vrac, la conversion du propène soit ≥ 90% en mole et la sélectivité de la formation d'acroléine, par rapport au propène transformé,
   soit ≥ 90% en mole,
   - que l'ordre dans le temps dans lequel le mélange gazeux réactionnel passe dans les zones de température A, B corresponde à l'ordre alphabétique des zones de température,
   - que la charge du catalyseur en lit fixe en vrac par le propène contenu dans le mélange réactionnel gazeux initial soit ≥ 90 Nl de propène/l de catalyseur en lit fixe en vrac * h, et
   - que la différence $T^{maxA}$-$T^{maxB}$, formée à partir de la température la plus élevée $T^{maxA}$ que le mélange gazeux réactionnel présente dans la zone de température A et de la température la plus élevée $T^{maxB}$ que le mélange gazeux réactionnel présente dans la zone de température B, soit ≥ 0°C, **caractérisé en ce que** la transition de la zone de température A dans la zone de température B dans catalyseur en lit fixe en vrac ne coïncide pas avec une transition d'une zone de catalyseur en lit fixe en vrac dans une autre zone de catalyseur en lit fixe en vrac.

2. Procédé selon la revendication 1, **caractérisé en ce que** $T^{maxA}$-$T^{maxB}$ est ≥ 3°C et ≤ 70°C.

3. Procédé selon la revendication 1, **caractérisé en ce que** $T^{maxA}$-$T^{maxB}$ est ≥ 20°C et ≤ 60°C.

4. Procédé selon la revendication 1 à 3, **caractérisé en ce que** la charge en propène du catalyseur en lit fixe en vrac soit ≥ 90 Nl/l * h et < 160 Nl/l * h.

5. Procédé selon l'une quelconque des revendications 1 à 3, **caractérisé en ce que** la charge en propène du catalyseur en lit fixe en vrac soit ≥ 160 Nl/l * h et ≤ 300 Nl/l * h.

6. Procédé selon l'une quelconque des revendications 1 à 5, **caractérisé en ce que** la température de la zone de réaction est de 305 à 365°C.

7. Procédé selon l'une quelconque des revendications 1 à 5, **caractérisé en ce que** la température de la zone de réaction est de 310 à 340°C.

8. Procédé selon l'une quelconque des revendications 1 à 7, **caractérisé en ce que** la zone de température A s'étend jusqu'à une conversion du propène de 50 à 70% en mole.

9. Procédé selon l'une quelconque des revendications 1 à 8, **caractérisé en ce que** la zone de température A s'étend jusqu'à une conversion du propène de 60 à 70% en mole.

10. Procédé selon l'une quelconque des revendications 1 à 9, **caractérisé en ce que** le rapport $O_2$:propène dans le mélange réactionnel gazeux initial vaut 1 à 2.

11. Procédé selon l'une quelconque des revendications 1 à 10, **caractérisé en ce que** la composition chimique de la

masse active utilisée sur la totalité du catalyseur en lit fixe en vrac est inchangée.

12. Procédé selon l'une quelconque des revendications 1 à 11, **caractérisé en ce que** la totalité du catalyseur en lit fixe en vrac ne comprend pas plus de quatre zones de catalyseur en lit fixe en vrac.

13. Procédé selon l'une quelconque des revendications 1 à 12, **caractérisé en ce que**, dans le cas d'une masse active uniforme sur la totalité du catalyseur en lit fixe en vrac, lors de la transition d'une zone de catalyseur en lit fixe en vrac dans une autre zone de catalyseur en lit fixe en vrac, dans le sens de l'écoulement du mélange gazeux réactionnel, la masse active volumique spécifique augmente d'au moins 5% en poids.

14. Procédé selon l'une quelconque des revendications 1 à 12, **caractérisé en ce que**, dans le cas d'une masse active uniforme sur la totalité du catalyseur en lit fixe en vrac, lors de la transition d'une zone de catalyseur en lit fixe en vrac dans une autre zone de catalyseur en lit fixe en vrac, dans le sens de l'écoulement du mélange gazeux réactionnel, la masse active volumique spécifique augmente d'au moins 10% en poids.

15. Procédé selon l'une quelconque des revendications 1 à 14, **caractérisé en ce que** dans le cas d'une masse active uniforme sur la totalité du catalyseur en lit fixe en vrac, la différence de la masse active volumique spécifique de la zone de catalyseur en lit fixe en vrac présentant l'activité volumique spécifique la plus basse et de celle de la zone de catalyseur en lit fixe en vrac présentant l'activité volumique spécifique la plus élevée n'est pas supérieure à 40% en poids.

16. Procédé selon l'une quelconque des revendications 1 à 15, **caractérisé en ce que** la dernière zone de catalyseur en lit fixe en vrac dans le sens de l'écoulement du mélange réactionnel gazeux est non diluée et n'est constituée que par des corps façonnés de catalyseur.

17. Procédé selon l'une quelconque des revendications 1 à 16, **caractérisé en ce que** la zone de catalyseur en lit fixe en vrac présentant l'activité volumique spécifique la plus élevée ne pénètre pas dans la zone de température A.

18. Procédé selon l'une quelconque des revendications 1 à 17, **caractérisé en ce qu'**il n'existe pas de transition d'une zone de catalyseur en lit fixe en vrac dans une autre zone de catalyseur en lit fixe en vrac dans le catalyseur en lit fixe en vrac dans la plage de $X \pm L * 4/100$, L étant la longueur du catalyseur en lit fixe en vrac et X l'endroit dans le catalyseur en lit fixe en vrac où se situe la transition de la zone de température A dans la zone de température B.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0053556 A
- DE 19948241 A
- DE 19910506 A
- DE 19945241 A
- EP 1106598 A
- DE 19955176 A
- DE 19948523 A
- DE 10101695 A
- DE 19948248 A
- DE 19955168 A
- EP 700714 A
- DE 10046957 A
- DE 10063162 A
- DE 3338380 C
- DE 19902562 A
- EP 15565 A
- DE 2380765 C
- EP 807465 A
- EP 279374 A
- DE 3300044 A
- EP 575897 A
- US 4438217 A
- DE 19855913 A
- WO 9824746 A
- DE 19746210 A
- JP 3294239 A
- EP 293224 A
- WO 02062737 A
- DE 4023239 A
- DE 2909671 A
- EP 293859 A
- EP 714700 A
- DE 2830765 C
- DE 2513405 C
- US 3147084 A
- DE 2201528 A
- EP 383224 A
- DE 2903218 A
- EP 700893 A
- EP 468290 B
- DE S2201528 A
- EP 382098 A
- DE 10302715 A
- DE 10261186 A
- WO 20219277 A
- WO 200219278 A
- WO 20219279 A
- EP 0214187 W
- EP 0214188 W
- EP 0214189 W
- EP 873783 A
- EP 1270065 A